# EUROPEAN PATENT APPLICATION

(11) **EP 4 732 863 A1**
(43) Date of publication of application: **29.04.2026**
(21) Application number: 23943289.1
(22) Date of filing: 08.11.2023
(51) Int. Cl.: A61M 1/36, A61M 1/16

(54) **INCLINED FLOW CHANNEL-BASED OXYGENATOR HAVING FEED LIQUID DISPERSION STRUCTURE**

(30) Priority: 25.06.2023 CN 202310746549
(71) Applicant: Jiangsusaitengyiliaokejiyouxiangongsi, Suzhou, Jiangsu 215123 (CN)
(72) Inventor: LIU, Peng, Suzhou, Jiangsu 215123 (CN); LIU, Ridong, Suzhou, Jiangsu 215123 (CN); LI, Zhengcai, Suzhou, Jiangsu 215123 (CN); SHI, Fangfang, Suzhou, Jiangsu 215123 (CN)
(74) Representative: Wang, Bo
(86) International application number: PCT/CN2023/130429
(87) International publication number: WO 2025/000810

(57) **Abstract**

The present invention discloses an oxygenator with obliquely traversing flow channels and a liquid infusion dispersion structure, including a housing and an oxygenation and temperature control module, wherein a water inlet, a water outlet, a blood inlet and a gas outlet are disposed at the bottom of the housing, and a gas inlet, a discharge port and a bleeding nozzle are disposed at the upper part; an oxygenation and temperature control module which is vertically arranged in the housing, wherein the oxygenation and temperature control module includes a mandrel, a temperature control membrane and an oxygen tension membrane successively from the inside to the outside; a lower end of the mandrel is directly opposite to the blood inlet; the temperature control membrane communicates with the water inlet and the water outlet; the oxygen tension membrane communicates with a gas inlet and a gas outlet; wherein the oxygenation and temperature control module further includes a liquid infusion dispersion structure; the liquid infusion dispersion structure includes a plurality of shunting steps disposed on an outer wall of the mandrel, and each of the shunting steps surrounds the outer wall of the mandrel, the plurality of the shunting steps being distributed at intervals along a long axis of the mandrel; and the diameter of the shunting steps close to a lower end of the mandrel is less than the diameter of the shunting steps close to an upper end of the mandrel. The present invention can shorten the precharge time and reduce the pressure loss of the oxygenator.

## Description

### TECHNICAL FIELD

The present invention relates to the technical field of medical devices, and more particularly, to an oxygenator with obliquely traversing flow channels and a liquid infusion dispersion structure.

### BACKGROUND

The extracorporeal circulation device for clinical operation is commonly known as the artificial lung or an oxygenator. The oxygenator, as a device for extracorporeal blood oxygenation, plays an important role in the extracorporeal circulation (CPB) and the extracorporeal membrane oxygenation (ECMO). In the process of application, its main function is to change the oxygen-poor venous blood into the oxygen-rich arterial blood and replace the lung function, so as to meet the needs of patients during operation.

Currently, the commercially available oxygenators for ECMO (hereinafter referred to as long-acting oxygenators) are designed in common with the oxygenators for cpb (hereinafter referred to as short-acting oxygenators) of the same brand, ignoring the fact that the long-acting oxygenators and the short-acting oxygenators have different focuses in actual use. It is mainly embodied as follows. (1) The short-acting oxygenators have sufficient preparation time. Thus, the requirements for rapid precharge and exhaust are not high, and the attention is paid to the oxygen supply after cardiac arrest. The gas exchange efficiency is more concerned. Most of the long-acting oxygenators are used in critical first-aid scenes in emergency, such as fulminant myocarditis, drowning, myocardial infarction and other conditions, which require the medical staff to quickly establish an ECMO circuit within the golden rescue window. Thus, they are more sensitive to the precharge time of the product. (2) Roller pumps are used in the short-acting oxygenators for most of the time, which causes insensitivity to the pressure loss of the oxygenator itself. All the long-acting oxygenators use centrifugal pumps to drive blood, which is more sensitive to the pressure loss of the oxygenators based on the characteristics of the centrifugal pumps. The pressure loss of the oxygenator determines the maximum output capacity of the pump and the speed difference at the same flow rate. The greater the pressure loss of the oxygenator itself is, the greater the speed is required to maintain the flow rate, thus aggravating the blood damage. In view of the above, it is necessary to design an oxygenator with short precharge time and small pressure loss to meet the functional needs in the critical first-aid scenes.

### SUMMARY

The technical problem to be solved by the embodiments of the present invention is to provide an oxygenator with obliquely traversing flow channels and a liquid infusion dispersion structure, which can shorten the precharge time of the oxygenator and reduce the pressure loss.

In order to solve the above-mentioned technical problem, the present invention provides an oxygenator with obliquely traversing flow channels and a liquid infusion dispersion structure, including:
a housing, wherein the bottom of the housing is provided with a water inlet, a water outlet, a blood inlet and a gas outlet; a gas inlet, a discharge port and a bleeding nozzle are provided at the upper part thereof; and
an oxygenation and temperature control module which is vertically disposed in the housing, wherein the oxygenation and temperature control module includes a mandrel, a temperature control membrane and an oxygen tension membrane successively from the inside to the outside; a lower end of the mandrel is directly opposite to the blood inlet; the temperature control membrane communicates with the water inlet and the water outlet; the oxygen tension membrane communicates with the gas inlet and the gas outlet;
wherein the oxygenation and temperature control module further includes a liquid infusion dispersion structure; the liquid infusion dispersion structure includes a plurality of shunting steps disposed on an outer wall of the mandrel, and each of the shunting steps surrounds the outer wall of the mandrel, the plurality of the shunting steps being distributed at intervals along a long axis of the mandrel; and the diameter of the shunting steps close to a lower end of the mandrel is less than the diameter of the shunting steps close to an upper end of the mandrel.

Optionally, the liquid infusion dispersion structure further includes a drainage member and a connecting rib; the drainage member is connected to the mandrel via the connecting rib; a first buffer space is disposed between a lower end surface of the mandrel and the drainage member; a second buffer space is disposed between the shunting step and the drainage member, and the second buffer space communicates with the first buffer space; and a drainage hole is disposed on the drainage member, and the drainage hole communicates with the blood inlet and the first buffer space.

Optionally, the lower end surface of the mandrel faces the drainage hole directly; and the blood entering the first buffer space is blocked by the lower end surface of the mandrel to disperse around the first buffer space.

Optionally, the plurality of connecting ribs are uniformly spaced apart on the outer wall of the mandrel; the plurality of connecting ribs divide the second buffer space into a plurality of subspaces;
the connecting rib is packaged by the temperature control membrane; the temperature control membrane is packaged by the oxygen tension membrane; and the blood flowing out of the first buffer space rises along the subspace and obliquely passes through the temperature control membrane and the oxygen tension membrane under the blocking action of the shunting steps.

Optionally, a step surface of the shunting step inclines from a lower end surface of the mandrel to an upper end surface of the mandrel.

Optionally, the step surface forms an included angle of 45°-90° with the long axis of the mandrel.

Optionally, the bleeding nozzle is lower than an effective contact height of the oxygen tension membrane; and the bleeding nozzle is inclined from a top of the housing to a bottom of the housing.

Optionally, the discharge port communicates with the oxygen tension membrane and is higher than the effective contact height of the oxygen tension membrane.

Optionally, the ratio of the outer diameter of the oxygen tension membrane to the effective contact height of the oxygen tension membrane is 1 : 1-2 : 1.

The practice of the present invention has the following beneficial effects.

The present disclosure designs a blood path and a mandrel structure of the oxygenator, so that blood enters the oxygenation and temperature control module from the blood inlet at the bottom of the housing. In the oxygenation and temperature control module, the liquid infusion dispersion structure disperses and conducts the blood, so that the blood obliquely passes through the temperature control membrane and the oxygen tension membrane, and then flows out from the bleeding nozzle at the upper part of the housing. In this design, the lower blood inlet mode is adopted, and the blood flow direction is consistent with the bubble direction, which is conducive to bubble flotation and discharge. In addition, by providing a plurality of shunting steps on the outer wall of the mandrel, after the blood enters the oxygenation and temperature control module from the blood inlet, during the lifting process of the blood liquid level, the blood is blocked by the shunting steps and uniformly dispersed outside to the temperature control membrane and the oxygen tension membrane, so that the temperature control membrane and the oxygen tension membrane at the lower part of the oxygenation and temperature control module are rapidly filled with blood, and the blood will drive the gas at the lower part of the oxygenation and temperature control module upwards while filling the lower part of the oxygenation and temperature control module, thereby speeding up the discharge and shortening the precharg time of the oxygenator. Furthermore, since the shunting steps shunt the blood, reducing the lifting speed of the blood liquid level and the blood flow rate, and the situation that the blood entrains air in the flow, i. e., the generation of air bubbles in the blood is reduced. The slow rising of the liquid level of the blood can further promote the smooth discharge of air in the oxygen tension membrane group. Furthermore, the blood is uniformly diffused to the periphery by the shunting steps, and the blood flow direction is changed so that the blood can obliquely passes through the temperature control membrane and the oxygen tension membrane, with a small pressure loss and a small damage to the blood. In the process of oblique passing, the angle between blood flow and gas flow direction is 45° -90°, which is beneficial to maintain the concentration difference between the blood side and the gas side, and can effectively improve the gas exchange rate and the oxygen exchange rate of the product.

### BRIEF DESCRIPTION OF DRAWINGS

Fig. 1 is a structurally schematic view of an oxygenator according to an embodiment of the present invention;
Fig. 2 is a side view of an oxygenator according to an embodiment of the present invention;
Fig. 3 is a front view of an oxygenator according to an embodiment of the present invention;
Fig. 4 is a cross-sectional view of an oxygenator according to an embodiment of the present invention;
Fig. 5 is a schematic view showing an overall structure of a mandrel and an infusion dispersion structure of an oxygenator according to an embodiment of the present invention;
Fig. 6 is a schematic view showing an overall structure of a mandrel and an infusion dispersion structure of an oxygenator according to an embodiment of the present invention;
Fig. 7 is a schematic diagram of the flow direction of blood, gas, and heating medium in an oxygenator according to an embodiment of the present invention.

In the drawings,
101 housing, 102 upper cover, 103 lower cover, 104 water inlet, 105 water outlet, 106 blood inlet, 107 gas outlet, 108 gas inlet, 109 discharge port, 110 bleeding nozzle, 111 shell, 201 mandrel, 202 temperature control membrane, 203 oxygen tension membrane, 204 shunting step, 205 drainage member, 206 drainage hole, 207 connecting rib, 208 first buffer space, 209 second buffer space, 210 subspace, 211 step surface, 301 first plugging layer, 302 second plugging layer, 401 gap.

### DETAILED DESCRIPTION

To make the above objects, features and advantages of the present invention more apparent, the detailed description of specific embodiments of the present invention will be made with reference to the accompanying drawings. In the following description, numerous specific details are set forth in order to provide a thorough understanding of the present invention. This invention may, however, be embodied in many different forms other than those described herein, and modifications may be made by those skilled in the art without departing from the spirit of the invention. Therefore, the invention is not limited to the specific embodiments disclosed below.

It will be understood that when an element is referred to as being "fixed" to another element, it can be directly on the other element or intervening elements may also be present. When an element is referred to as being "connected" to another element, it can be directly connected to the other element or intervening elements may also be present. The terms "vertical", "horizontal", "left", "right", and the like are used herein for illustrative purposes only.

Unless defined otherwise, all technical and scientific terms used herein have the same meaning as commonly understood by one of ordinary skill in the art to which this invention belongs. The terminology used in the description of the invention herein is for the purpose of describing particular examples only and is not intended to be limiting of the invention. As used herein, the term "and/or" includes any and all combinations of one or more of the associated listed items.

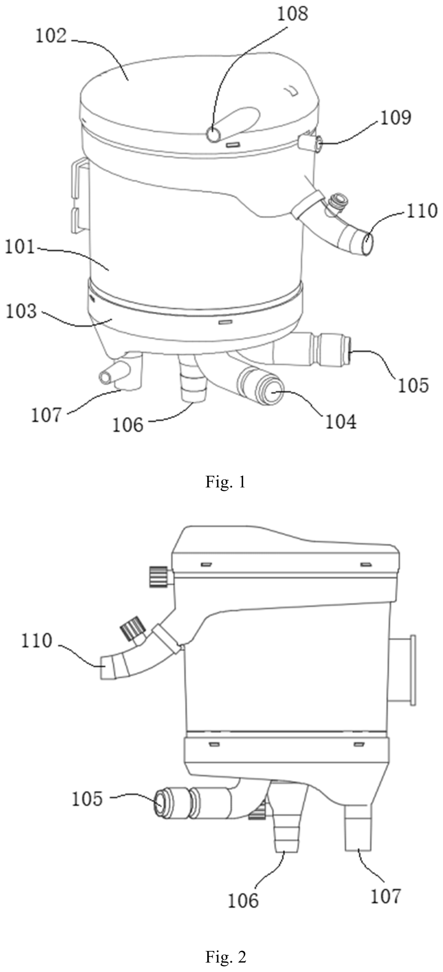

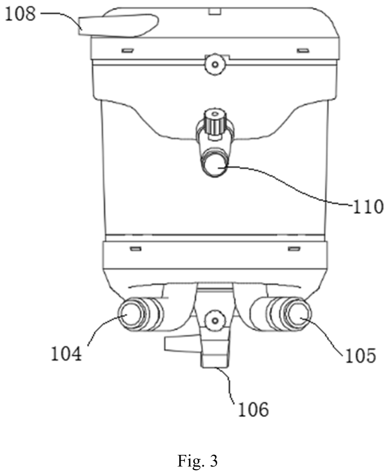

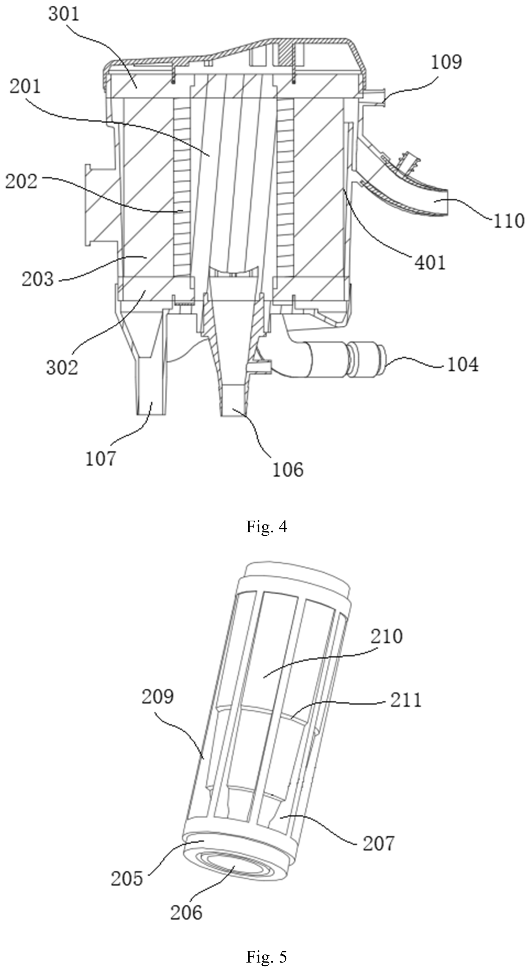

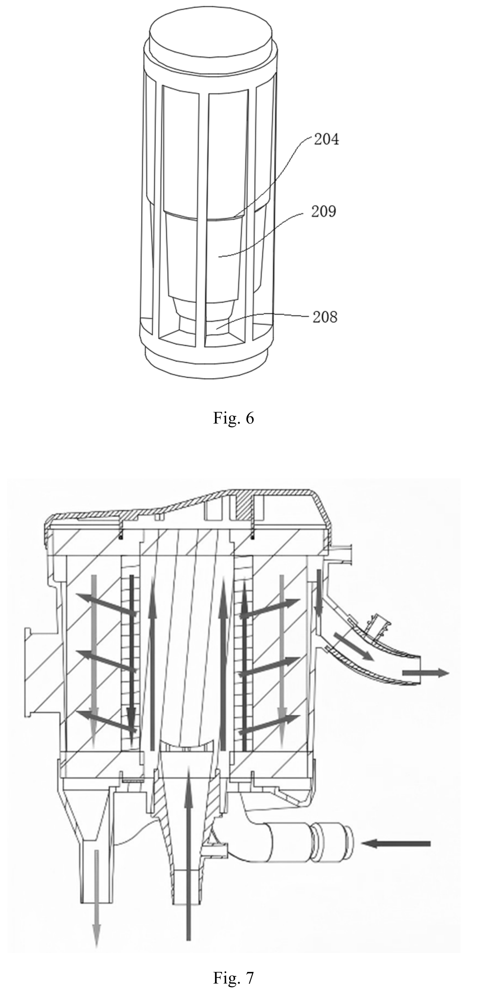

The present embodiment provides an oxygenator with obliquely traversing flow channels and a liquid infusion dispersion structure, including a housing 101 and an oxygenation and temperature control module. Referring to Figs. 1 to 4, a water inlet 104, a water outlet 105, a blood inlet 106 and a gas outlet 107 are disposed at the bottom of the housing 101, and a gas inlet 108, a discharge port 109 and a bleeding nozzle 110 are provided at the upper part thereof. The oxygenation and temperature control module is vertically disposed in the housing 101. The oxygenation and temperature control module includes a mandrel 201, a temperature control membrane 202 and an oxygen tension membrane 203 successively from the inside to the outside. A lower end of the mandrel 201 is directly opposite to the blood inlet 106. The temperature control membrane 202 communicates with the water inlet 104 and the water outlet 105. The oxygen tension membrane 203 communicates with the gas inlet 108 and the gas outlet 107. With reference to Figs. 5 and 6, the oxygenation and temperature control module further includes a liquid infusion dispersion structure. The liquid infusion dispersion structure includes a plurality of shunting steps 204 disposed on the outer wall of the mandrel 201, and each of the shunting steps 204 surrounds the outer wall of the mandrel 201, the plurality of shunting steps 204 being distributed at intervals along the long axis of the mandrel 201. The diameter of the shunting steps 204 close to the lower end of the mandrel 201 is less than the diameter of the shunting steps 204 close to the upper end of the mandrel 201. Here, all the shunting steps 204 are coaxial with the mandrel 201. The shunting steps 204 are annular. The width of each region on a single shunting step 204 is the same. The width of each shunting step 204 can be the same or different. The width of the shunting steps 204 refers to the height of the shunting steps 204 protruding from the outer wall of the mandrel 201.

In a possible implementation, the housing 101 includes a shell 111, an upper cover 102 disposed at an opening of a top of the shell and a lower cover 103 disposed at an opening of a bottom of the shell 111; the gas inlet 108 is disposed on the upper cover 102; the discharge port 109 and the bleeding nozzle 110 are disposed on the shell 111 at a position close to the upper cover 102; and the water inlet 104, the water outlet 105, the blood inlet 106 and the gas outlet 107 are disposed on the lower cover 103. The oxygenation and temperature control module is disposed in the shell; a first plugging layer 301 is provided between an upper end of the oxygenation and temperature control module and the upper cover 102; a second plugging layer 302 is provided between a lower end of the oxygenator module and the lower cover 103; a mandrel 201 is vertically disposed in the middle part of the shell, and the upper end of the mandrel 201 is fixed to the first plugging layer 301; a drainage member 205 is connected to the blood inlet 106; the temperature control membrane 202 passes through the second plugging layer 302 and communicates with the water inlet 104 and the water outlet 105; the blood inlet 106 passes through the second plugging layer 302 and communicates with the drainage hole 206; the inlet of the oxygen tension membrane 203 passes through the first plugging layer 301 and communicates with the gas inlet 108; and the outlet of the oxygen tension membrane 203 passes through the second plugging layer 302 and communicates with the gas outlet 107.

Fig. 7 shows the flow directions of blood, heating medium, and oxygen in the oxygenator, where red represents blood, blue represents heating medium, and green represents oxygen. Herein, oxygen gas enters from the gas inlet 108 into the interior of the oxygen tension membrane filament filling the oxygen tension membrane 203. The oxygen gas in the oxygen tension membrane 203 exchanges with carbon dioxide in the blood, and then the carbon dioxide is carried out by the oxygen tension membrane 203 and discharged from the gas outlet 107. The heating medium, such as water, enters the interior of the temperature control membrane filament filled with the temperature control membrane 202 from the water inlet 104. The heat exchange occurs when the blood flows through the temperature control membrane 202 so as to raise the temperature of the blood. The heating medium in the temperature control membrane 202 can be discharged from the water outlet 105, and re-enters the temperature control membrane 202 after being heated externally. From the blood inlet 106, the blood enters a space between the mandrel 201 and the temperature control membrane 202, disperses around under the drainage effect of the liquid infusion dispersion structure, obliquely passes through the temperature control membrane 202 and the oxygen tension membrane 203, and then flows out from the bleeding nozzle 110.

The present disclosure designs a blood path and a mandrel 201 structure of the oxygenator, so that blood enters the oxygenation and temperature control module from the blood inlet 106 at the bottom of the housing 101. In the oxygenation and temperature control module, the liquid infusion dispersion structure disperses and conducts the blood, so that the blood obliquely passes through the temperature control membrane 202 and the oxygen tension membrane 203, and then flows out from the bleeding nozzle 110 at the upper part of the housing 101. The liquid infusion dispersion structure is designed to reduce the blood pressure loss as well as the precharge time of the oxygenator. It is mainly embodied as follows.
1. The lower blood inlet mode is adopted, and the blood flow direction is consistent with the bubble direction, which is conducive to bubble flotation and discharge.
2. By providing a plurality of shunting steps 204 on the outer wall of the mandrel 201, after the blood enters the oxygenation and temperature control module from the blood inlet 106, during the lifting process of the blood liquid level, the blood is blocked by the shunting steps 204 and uniformly dispersed outside to the temperature control membrane 202 and the oxygen tension membrane 203, so that the temperature control membrane 202 and the oxygen tension membrane 203 at the lower part of the oxygenation and temperature control module are rapidly filled with blood. The gas at the lower part of the oxygenation and temperature control module is driven upwards, thereby speeding up gas discharge and shortening the precharge time of the oxygenator;
3. The shunting step 204 distributes the blood evenly to the periphery, expands the contact area between the blood and the temperature control membrane 202 and the oxygen tension membrane 203 at the initial stage of blood injection, and improves the oxygenation efficiency.
4. Since the shunting step 204 shunts the blood, the lifting speed of the liquid surface of the blood is reduced, and the situation where the blood entrains air in the flow is reduced, i. e., the generation of air bubbles in the blood is reduced. The slow lifting of the liquid surface of the blood further promotes the smooth discharge of air in the oxygen tension membrane 203 group from the discharge port 109.
5. The blood diffuses uniformly to the periphery by the shunting step 204, and the blood flow direction is changed so that the blood can obliquely pass through the temperature control membrane 202 and the oxygen tension membrane 203. The passing ability is good, the pressure loss is small, and the blood damage is small.

With reference to Figs. 5 and 6, the liquid infusion dispersion structure further includes a drainage member 205 and a connecting rib 207; the drainage member 205 is connected to the mandrel 201 via the connecting rib 207; a first buffer space 208 is disposed between a lower end surface of the mandrel 201 and the drainage member 205; a second buffer space 209 is disposed between the shunting step 204 and the drainage member 205, and the second buffer space 209 communicates with the first buffer space 208; and a drainage hole 206 is disposed on the drainage member 205, and the drainage hole 206 communicates with the blood inlet 106 and the first buffer space 208. After entering from the blood inlet 106, the blood firstly resides in the first buffer space 208. In this process, the blood diffuses to the periphery and enters the lower part of the temperature control membrane 202 and the oxygen tension membrane 203, and the air in the lower part of the shell is driven upwards. With the continuous injection of the subsequent blood, the blood does not pass through the first buffer space 208 and then rises to the second buffer space 209. The blood continues to flow upwards in the second buffer space 209, and is blocked by the shunting step 204, diffuses to the periphery and enters the middle part of the temperature control membrane 202 and the oxygen tension membrane 203. The air in the housing is driven upwards from the bottom, so that the air in the oxygenator is rapidly discharged, and the precharge time is shortened. Furthermore, the blood enters from the blood inlet 106 at the bottom of the oxygenator, obliquely passes through the temperature control membrane 202 and the oxygen tension membrane 203, and then flows out from the blood inlet at the upper part of the oxygenator, and the blood flow direction is consistent with the gas flow direction, which conforms to the physical characteristics of bubbles in the liquid, so as to facilitate the natural and rapid discharge of the gas, without the need for the user to strike the housing 101 to facilitate the discharge of the gas. By experiments, the connected oxygenator of this embodiment can complete the gas discharge in 15 seconds, while the discharge action of the conventional oxygenator takes 1-5 minutes (depending on the proficiency of the operator). It can be seen that the oxygenator of this embodiment greatly shortens the precharge time and also reduces the requirements of the product for the operator.

In a possible implementation, the lower end surface of the mandrel 201 faces the drainage hole 206 directly; and the blood entering the first buffer space 208 is blocked by the lower end surface of the mandrel 201 to disperse around the first buffer space 208. The lower end face of the mandrel 201 faces the drainage hole 206 directly. The blood directly hits the lower end face of the mandrel 201 after exiting from the drainage hole 206, and disperses around under the restriction of the lower end face of the mandrel 201, which is beneficial to the uniform diffusion of the blood at the lower part of the oxygenator, quickly squeezing out the air at the lower part of the oxygenator, and improving the utilization rate of the temperature control membrane 202 and the oxygen tension membrane 203 at the initial stage of the blood injection.

In a possible implementation, the plurality of connecting ribs 207 are uniformly spaced apart on the outer wall of the mandrel 201. The plurality of connecting ribs 207 divide the second buffer space 209 into a plurality of subspaces 210. The connecting rib 207 is packaged by the temperature control membrane 202. The temperature control membrane 202 is packaged by the oxygen tension membrane 203. The blood flowing out of the first buffer space 208 flows into each subspace 210 uniformly, and rises slowly in the subspace 210. In the rising process, the blood is blocked by the shunting step 204 and diffuses to the periphery, and then obliquely passes through the temperature control membrane 202 and the oxygen tension membrane 203. The step surface 211 of the shunting step 204 inclines from the lower end surface of the mandrel 201 to the upper end surface of the mandrel 201. Specifically, the step surface 211 forms an angle of 45°-90° with the long axis of the mandrel 201.

The temperature control membrane 202 includes a plurality of temperature control membrane filaments distributed along the long axis of the mandrel 201. A flow gap is formed between adjacent temperature control membrane filaments, and a plurality of flow gaps form a louver shape in the radial direction of the oxygenator. The oxygen tension membrane 203 is wrapped around the temperature control membrane 202, and includes a plurality of oxygen tension membrane filaments distributed along the long axis of the mandrel 201 and staggered with each other. A through-flow hole is formed among adjacent oxygen tension membrane filaments, and the plurality of through-flow holes form a honeycomb shape in the radial direction of the oxygenator.

Since the step surface 211 forms an included angle of 45°-90° with the long axis of the mandrel 201, after encountering the step surface 211, the blood is switched from a vertical upward flow direction to an oblique upward flow direction so as to pass through the flow gap obliquely upwards. The flow direction of blood obliquely passing through the oxygen tension membrane is similar to the step shape, and it can be divided into two states: transversing the flow hole and flowing along the oxygen tension membrane filament. Specifically, since the flow gap is an elongated channel and the through-flow hole is a hole-shaped channel, when the blood passes through the flow gap and is blocked by the oxygen tension membrane filament, the blood will switch from the obliquely upward flow direction to the transverse flow direction to transverse the through-flow hole adjacent to the temperature control membrane. Then, driven by the continuously injected blood, the blood will flow upwards along the oxygen tension membrane filament to transverse the through-flow hole adjacent to the inner wall of the shell, and will not flow out of the bleeding nozzle until it reaches the upper part of the bleeding nozzle. Herein, "transverse" means that the blood passes through the through-flow hole in a manner perpendicular to the through-flow hole, i. e., the blood flows in a radial direction with the oxygenator, and in this state, the blood is subject to a small resistance, with a fast flow, and a small damage to the blood.

In this embodiment, the blood enters the vicinity of the mandrel and disperses to the periphery under the restriction of the lower end face of the mandrel, the shunting steps and the connecting ribs, which belongs to a first shunting. Then, the flow gap disperses the blood into a smaller stream, which belongs to a second shunting. After the two shunts, the blood is uniformly dispersed, on the one hand, facilitating the extrusion of the air in the shell; on the other hand, making the blood contact with the oxygen tension membrane filaments more sufficient, improving the oxygenation effect, and improving the utilization rate of the oxygen tension membrane at the initial stage of the blood injection. In the temperature control membrane, the flow area of the flow gap is larger. The blood obliquely passing through the temperature control membrane is faster in speed and less hindered, reaching the oxygen tension membrane quickly through the temperature control membrane. In the oxygen tension membrane, the blood transverses the flow-through hole, which has the advantages of low flow resistance and small pressure loss. The blood flows along the membrane filaments of the oxygen tension membrane to increase the contact area between the blood and the oxygen tension membrane, so as to enhance the oxygenation effect. As can be seen, this embodiment combines the performance of low transversing-flow resistance with concurrent flow enhancement of oxygenation.

In the present embodiment, the ratio of the outer diameter of the oxygen tension membrane 203 to the effective contact height of the oxygen tension membrane 203 may be 1 : 1-2 : 1 based on the difference in the maximum flow rate of the diagonal design. The effective contact height of the oxygen tension membrane 203 refers to the height of the portion of the oxygen tension membrane 203 within the shell that can convert oxygen-poor blood to oxygen-rich blood, not exceeding the discharge port 109. The effective contact height of the oxygen tension membrane 203 may specifically be the distance from the top surface of the second plugging layer 302 to the lower end surface of the discharge port 109.

Referring to Fig. 4, the discharge port 109 communicates with the oxygen tension membrane 203 and is higher than the effective contact height of the oxygen tension membrane 203. Air bubbles floating up to the blood level can be discharged through the discharge port 109, but blood does not flow out of the discharge port 109. The bleeding nozzle 110 communicates with the oxygen tension membrane 203, the inlet of the bleeding nozzle 110 is lower than the effective contact height of the oxygen tension membrane 203, and the bleeding nozzle 110 is inclined from the top of the housing 101 to the bottom of the housing 101. Referring to Fig. 7, there is a gap between the oxygen tension membrane 203 and the inner wall of the shell. The width of the gap increases from the bottom of the shell to the top of the shell, and the inlet of the bleeding nozzle 110 communicates with the upper part of the gap. The blood is collected at the gap after passing through the oxygen tension membrane 203, and then flows out from the bleeding nozzle 110. When the blood flows from the gap to the bleeding nozzle 110, the flow direction is nearly vertical downward. Since the bleeding nozzle 110 is inclined downward, the blood turning angle is small and the change in flow velocity is small, so that the blood pressure loss and blood damage can be reduced. The blood pressure loss can be further reduced by using a smooth elbow design.

the flow of blood in the oxygenator of this embodiment is simulated for calculation by CFD. Most of the blood flow can traverse the oxygen tension membrane filaments during the flow. The pressure loss of the oxygenator is reduced. The smaller the pressure loss is, the lower the rotation speed of the centrifugal pump is at the same flow rate, and the smaller the blood damage is. The pressure loss measured at the maximum flow rate of the oxygenators of the embodiments is ≤ 70 mmHg, which is lower than the pressure losses of all the oxygenators known to date. Meanwhile, the unique curved, obliquely downward design of the nozzle 110 of the present embodiment also effectively reduces pressure loss and blood damage and avoids the blood damage associated with conventional horizontal outlets.

The technical features of the above-mentioned embodiments can be combined in any combination. In order to make the description concise, not all the possible combinations of the technical features in the above-mentioned embodiments are described. However, as long as there is no contradiction between the combinations of the technical features, they should be considered as the scope of disclosure contained in the present description.

The embodiments described above represent only a few implementations of the invention and are described in more detail and should not be construed as limiting the scope of the invention. It should be noted that several variations and modifications can be made by one skilled in the art without departing from the inventive concept, which is within the scope of the present invention. Accordingly, the protection sought herein is as set forth in the claims below.

## Claims

1. An oxygenator with obliquely traversing flow channels and a liquid infusion dispersion structure, **characterized by** comprising:
a housing (101), wherein the bottom of the housing (101) is provided with a water inlet (104), a water outlet (105), a blood inlet (106) and a gas outlet (107); the upper part is provided with a gas inlet (108), a discharge port (109) and a bleeding nozzle (110); and
an oxygenation and temperature control module which is vertically disposed in the housing (101), wherein the oxygenation and temperature control module comprises a mandrel (201), a temperature control membrane (202) and an oxygen tension membrane (203) successively from the inside to the outside; a lower end of the mandrel (201) is directly opposite to the blood inlet (106); the temperature control membrane (202) communicates with the water inlet (104) and the water outlet (105); the oxygen tension membrane (203) communicates with the gas inlet (108) and the gas outlet (107);
wherein the oxygenation and temperature control module further comprises a liquid infusion dispersion structure; the liquid infusion dispersion structure comprises a plurality of shunting steps (204) disposed on an outer wall of the mandrel (201), and each of the shunting steps (204) surrounds the outer wall of the mandrel (201), the plurality of the shunting steps (204) being distributed at intervals along a long axis of the mandrel (201); and the diameter of the shunting steps (204) close to a lower end of the mandrel (201) is less than the diameter of the shunting steps (204) close to an upper end of the mandrel (201).

2. The oxygenator according to claim 1, **characterized in that**
the liquid infusion dispersion structure further comprises a drainage member (205) and a connecting rib (207); the drainage member (205) is connected to the mandrel (201) via the connecting rib (207); a first buffer space (208) is disposed between a lower end surface of the mandrel (201) and the drainage member (205); a second buffer space (209) is disposed between the shunting step (204) and the drainage member (205), and the second buffer space (209) communicates with the first buffer space (208); and a drainage hole (206) is disposed on the drainage member (205), and the drainage hole (206) communicates with the blood inlet (106) and the first buffer space (208).

3. The oxygenator according to claim 2, **characterized in that**
the lower end surface of the mandrel (201) faces the drainage hole (206) directly; and the blood entering the first buffer space (208) is blocked by the lower end surface of the mandrel (201) to disperse around the first buffer space (208).

4. The oxygenator according to claim 2, **characterized in that**
the plurality of connecting ribs (207) are uniformly spaced apart on the outer wall of the mandrel (201); the plurality of connecting ribs (207) divide the second buffer space (209) into a plurality of subspaces (210);
the connecting rib (207) is packaged by the temperature control membrane (202); the temperature control membrane (202) is packaged by the oxygen tension membrane (203); and the blood flowing out of the first buffer space (208) rises along the subspace (210) and obliquely passes through the temperature control membrane (202) and the oxygen tension membrane (203) under the blocking action of the shunting steps (204).

5. The oxygenator according to claim 4, **characterized in that**
a step surface (211) of the shunting step (204) inclines from a lower end surface of the mandrel (201) to an upper end surface of the mandrel (201).

6. The oxygenator according to claim 5, **characterized in that**
the step surface (211) forms an included angle of 45°-90° with the long axis of the mandrel (201).

7. The oxygenator according to claim 1, **characterized in that**
the bleeding nozzle (110) is lower than an effective contact height of the oxygen tension membrane (203); and the bleeding nozzle (110) is inclined from a top of the housing (101) to a bottom of the housing (101).

8. The oxygenator according to claim 7, wherein the bleeding nozzle (110) is a curved tube;
the discharge port (109) communicates with the oxygen tension membrane (203) and is higher than the effective contact height of the oxygen tension membrane (203).

9. The oxygenator according to claim 5, **characterized in that**
the ratio of the outer diameter of the oxygen tension membrane (203) to the effective contact height of the oxygen tension membrane (203) is 1 : 1-2 : 1.

10. The oxygenator according to claim 1, **characterized in that**
the housing (101) comprises a shell (111), an upper cover (102) disposed at an opening of a top of the shell and a lower cover (103) disposed at an opening of a bottom of the shell (111); the gas inlet (108) is disposed on the upper cover (102); the discharge port (109) and the bleeding nozzle (110) are disposed on the shell (111) at a position close to the upper cover (102); the water inlet (104), the water outlet (105), the blood inlet (106) and the gas outlet (107) are disposed on the lower cover (103);
the oxygenation and temperature control module is disposed in the shell; a first plugging layer (301) is provided between an upper end of the oxygenation and temperature control module and the upper cover (102); a second plugging layer (302) is provided between a lower end of the oxygenator module and the lower cover (103); the temperature control membrane (202) passes through the second plugging layer (302) and communicates with the water inlet (104) and the water outlet (105); the blood inlet (106) passes through the second plugging layer (302) and communicates with the drainage hole (206); the inlet of the oxygen tension membrane (203) passes through the first plugging layer (301) and communicates with the gas inlet (108); and the outlet of the oxygen tension membrane (203) passes through the second plugging layer (302) and communicates with the gas outlet (107).
